# EUROPEAN PATENT APPLICATION

(11) **EP 3 295 884 A1**
(43) Date of publication of application: **21.03.2018**
(21) Application number: 16900398.5
(22) Date of filing: 26.04.2016
(51) Int. Cl.: A61B 18/14

(54) **ENERGY TREATMENT TOOL, TREATMENT SYSTEM, AND CONTROL DEVICE**

(71) Applicant: Olympus Corporation, Hachioji-shi, Tokyo 192-8507 (JP)
(72) Inventor: HAYASHIDA, Tsuyoshi, Hachioji-shi Tokyo 192-8507 (JP); SAKAO, Satomi, Hachioji-shi Tokyo 192-8507 (JP)
(74) Representative: Gunzelmann, Rainer
(86) International application number: PCT/JP2016/063091
(87) International publication number: WO 2017/187525

(57) **Abstract**

An energy treatment instrument includes a first grasping piece, and a second grasping piece opening and closing relative to the first grasping piece and grasping a treated target between the first grasping piece and the second grasping piece. An actuation state of the energy treatment instrument is switched between a first mode in which the treated target is coagulated when a forceps does not exist in a predetermined range from a position where the treated target is grasped, and a second mode in which the treated target is coagulated when the forceps exists in the predetermined range.

## Description

### Technical Field

The present invention relates to an energy treatment instrument which applies treatment energy to a treated target grasped between a pair of grasping pieces, a treatment system including the energy treatment instrument, and a control device for use in combination with the energy treatment instrument.

### Background Art

PCT International Publication No. 2012/061638 discloses an energy treatment instrument which grasps a treated target, such as a biological tissue, between a pair of grasping pieces. In this energy treatment instrument, the grasping pieces are provided with electrodes, respectively. By electric energy being supplied to both electrodes, a high-frequency current flows between the electrodes through the grasped treated target. Thereby, the high-frequency current is applied as treatment energy to the treated target.

### Summary of Invention

When treatment is performed by using the energy treatment instrument as disclosed in PCT International Publication No. 2012/061638, there is a case in which in the vicinity of a region where a blood vessel is clamped by a forceps that is separate from the energy treatment instrument, the blood vessel is grasped by the paired grasping pieces, and the grasped blood vessel is sealed. In such treatment, if treatment is performed in the same manner as in the case in which the blood vessel is grasped in a position apart from the forceps, there is possibility that the treatment of sealing the blood vessel by using the treatment energy is affected. Consequently, there is a possibility that the sealing performance of the blood vessel, such as a pressure resistance value of the sealed blood vessel (difficulty in blood flow), is affected.

The present invention has been made in order to solve the above problem. The object of the invention is to provide a treatment system and a control device, which can exhibit a proper treatment performance even when a treated target is grasped near a region which is clamped by a forceps.

To solve the above mentioned problems, according to one aspect of the invention, an energy treatment instrument including a first grasping piece, and a second grasping piece configured to open and close relative to the first grasping piece and configured to grasp a treated target between the first grasping piece and the second grasping piece, wherein an actuation state is switched between a first mode in which the treated target is coagulated when a forceps does not exist in a predetermined range from a position where the treated target is grasped, and a second mode in which the treated target is coagulated when the forceps exists in the predetermined range.

According to one another aspect of the invention, a control device for use in combination with an energy treatment instrument including a first grasping piece, and a second grasping piece configured to open and close relative to the first grasping piece and configured to grasp a treated target between the first grasping piece and the second grasping piece, the control device including: an energy output source configured to output electric energy which is supplied to the energy treatment instrument, and configured to apply treatment energy to the treated target which is grasped between the first grasping piece and the second grasping piece, by the electric energy being supplied to the energy treatment instrument; and a processor configured to judge whether a forceps exists in a predetermined range from a position where the treated target is grasped, based on an observation image observed by an observation element, and configured to execute at least one of controlling an output of the electric energy from the energy output source, based on a judgement result of the forceps, and increasing a grasping force of the treated target between the first grasping piece and the second grasping piece in a case in which it was judged that the forceps exists, compared to a case in which it was judged that the forceps does not exist.

### Brief Description of Drawings

FIG. 1 is a schematic view illustrating a treatment system according to a first embodiment;
FIG. 2 is a block diagram illustrating a control configuration in the treatment system according to the first embodiment;
FIG. 3 is a flowchart illustrating a process in a processor in a seal treatment of a blood vessel using the treatment system according to the first embodiment;
FIG. 4 is a flowchart illustrating a process in output control in a first seal mode of the processor according to the first embodiment;
FIG. 5 is a schematic view illustrating an example of a variation with time of an impedance between a pair of grasping pieces, in a state in which the processor according to the first embodiment is executing output control in each of the first seal mode and second seal mode;
FIG. 6 is a schematic view illustrating an example of an observation image in a state in which a blood vessel is grasped between the grasping pieces near a region clamped by a forceps in the first embodiment;
FIG. 7 is a schematic view illustrating an example of a variation with time of an impedance between a pair of grasping pieces, in a state in which a processor according to a first modification of the first embodiment is executing output control in each of the first seal mode and second seal mode;
FIG. 8 is a flowchart illustrating a process in output control in the second seal mode of a processor according to a second modification of the first embodiment;
FIG. 9 is a schematic view illustrating an example of a variation with time of an impedance between a pair of grasping pieces, in a state in which a processor according to the second modification of the first embodiment is executing output control in each of the first seal mode and second seal mode;
FIG. 10 is a flowchart illustrating a process in output control in the second seal mode of a processor according to a third modification of the first embodiment;
FIG. 11 is a flowchart illustrating a process in a processor in a seal treatment of a blood vessel using a treatment system according to a fourth modification of the first embodiment;
FIG. 12 is a block diagram illustrating a control configuration in a treatment system according to a second embodiment;
FIG. 13 is a schematic view illustrating an example of a grasping force adjustment element according to the second embodiment.
FIG. 14 is a flowchart illustrating a process in a processor in a seal treatment of a blood vessel using the treatment system according to the second embodiment; and
FIG. 15 is a flowchart illustrating a process in a processor in a seal treatment of a blood vessel using a treatment system according to one modification of the first embodiment and second embodiment.

### Description of Embodiments

### (First Embodiment)

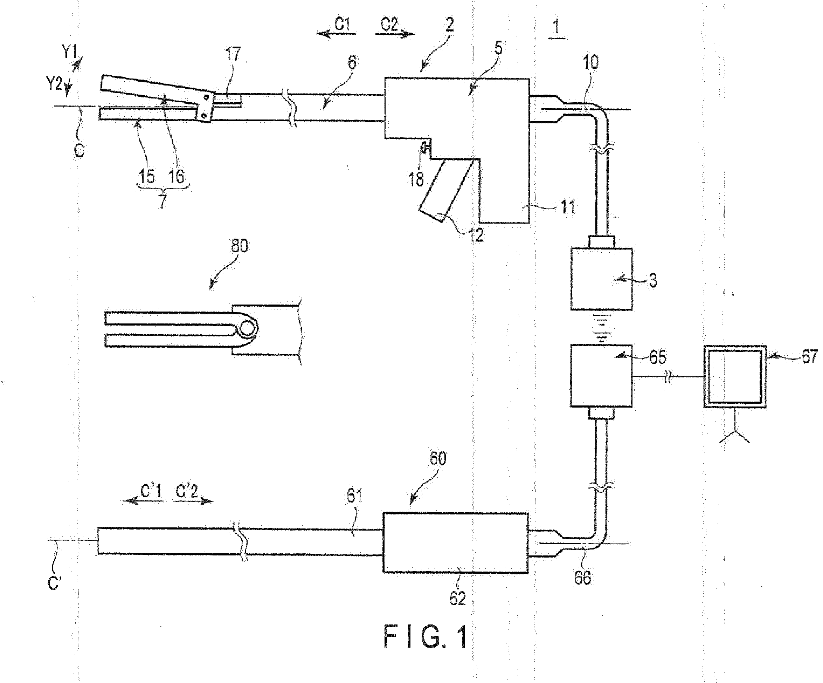

A first embodiment of the present invention will be described with reference to FIG. 1 to FIG. 6. FIG. 1 is a view illustrating a treatment system 1 according to the present embodiment. As illustrated in FIG. 1, the treatment system 1 includes an energy treatment instrument 2 and a control device (energy control device) 3. The energy treatment instrument 2 has a longitudinal axis C. Here, in the energy treatment instrument 2, one side of a direction along the longitudinal axis C is defined as a distal side (arrow C1 side), and a side opposite to the distal side is defined as a proximal side (arrow C2 side).

The energy treatment instrument 2 includes a housing 5 which can be held, a sheath (shaft) 6 which is coupled to the distal side of the housing 5, and an end effector 7 which is provided in a distal portion of the sheath 6. One end of a cable 10 is connected to the housing 5 of the energy treatment instrument 2. The other end of the cable 10 is detachably connected to the control device 3. In addition, the housing 5 is provided with a grip (stationary handle) 11. A handle (movable handle) 12 is rotatably attached to the housing 5. By the handle 12 rotating relative to the housing 5, the handle 12 opens or closes relative to the grip 11. In the present embodiment, the handle 12 is located on the distal side with respect to the grip 11, and the handle 12 moves substantially in parallel to the longitudinal axis C in the motion of opening or closing relative to the grip 11. However, the embodiment is not limited to this. For instance, in one example, the handle 12 may be located on the proximal side with respect to the grip 11. In another example, the handle 12 may be located on a side opposite to the grip 11 with respect to the longitudinal axis C, and a movement direction in the motion of opening or closing relative to the grip 11 may cross (may be substantially perpendicular to) the longitudinal axis C.

The sheath 6 extends along the longitudinal axis C. In addition, the end effector 7 includes a first grasping piece 15, and a second grasping piece 16 which closes or opens relative to the first grasping piece 15. The handle 12 and end effector 7 are coupled via a movable member 17 which extends along the longitudinal axis C in the inside of the sheath 6. The handle 12, which is an opening and closing operation input section, is opened or closed relative to the grip 11. Thereby, the movable member 17 moves along the longitudinal axis C relative to the sheath 6 and housing 5, and the pair of grasping pieces 15 and 16 open or close relative to each other. By the grasping pieces 15 and 16 closing relative to each other, the grasping pieces 15 and 16 grasp a biological tissue, such as a blood vessel, as a treated target. The opening and closing directions (directions of arrow Y1 and arrow Y2) of the grasping pieces 15 and 16 cross (are substantially perpendicular to) the longitudinal axis C.

It should suffice if the end effector 7 is configured such that the paired grasping pieces 15 and 16 open or close relative to each other in accordance with an opening operation or a closing operation of the handle 12. For instance, in one example, one of the grasping pieces 15 and 16 is formed integral with the sheath 6 or fixed to the sheath 6, and the other of the grasping pieces 15 and 16 is rotatably attached to the distal portion of the sheath 6. In another example, both the grasping pieces 15 and 16 are rotatably attached to the distal portion of the sheath 6. In still another example, a rod member (not shown) is inserted through the sheath 6, and one of the grasping pieces 15 and 16 is formed by a projecting portion of the rod member (probe), which projects from the sheath 6 toward the distal side. In addition, the other of the grasping pieces 15 and 16 is rotatably attached to the distal portion of the sheath 6. Besides, in one example, a rotary operation knob (not shown) may be attached to the housing 5. In this case, by rotating the rotary operation knob relative to the housing 5, the sheath 6 and end effector 7 rotate, together with the rotary operation knob, around the longitudinal axis C relative to the housing 5. Thereby, the angular position of the end effector 7 around the longitudinal axis C is adjusted.

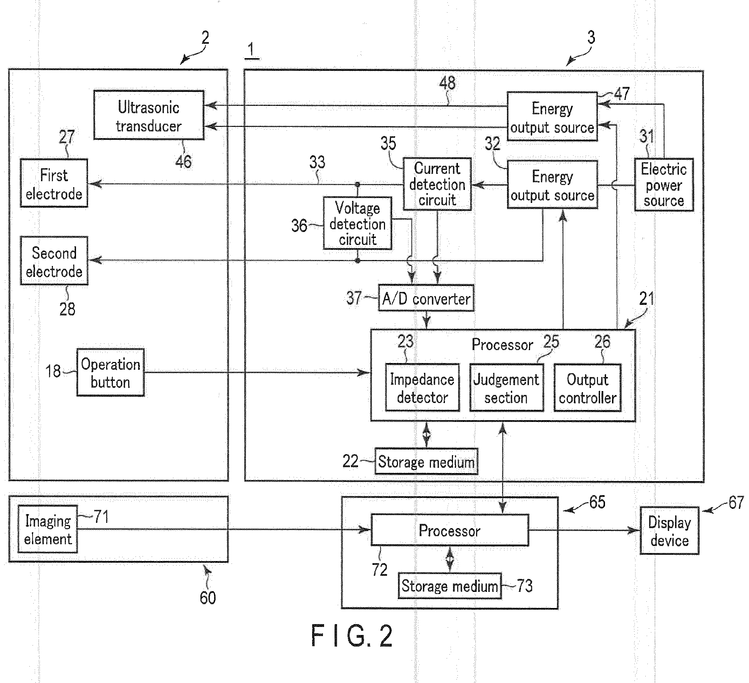

FIG. 2 is a view illustrating a control configuration in the treatment system 1. As illustrated in FIG. 2, the control device 3 includes a processor (controller) 21 which controls the entirety of the treatment system 1, and a storage medium 22. The processor 21 is formed of an integrated circuit including a CPU (Central Processing Unit), an ASIC (Application Specific Integrated Circuit) or an FPGA (Field Programmable Gate Array). The processor 21 may be formed of a single integrated circuit, or may be formed of a plurality of integrated circuits. The process in the processor 21 is executed according to a program stored in the processor 21 or storage medium 22. In addition, the storage medium 22 stores a processing program which is used in the processor 21, and parameters, tables, etc. which are used in arithmetic operations in the processor 21. The processor 21 includes an impedance detector 23, a judgement section 25 and an output controller 26. The impedance detector 23, judgement section 25 and output controller 26 function as parts of the processor 21, and execute some of the processes which are executed by the processor 21.

In the end effector 7 of the energy treatment instrument 2, the first grasping piece 15 is provided with a first electrode 27, and the second grasping piece 16 is provided with a second electrode 28. The electrodes 27 and 28 are formed of an electrically conductive material. The control device 3 includes an electric power source 31 which is a battery or a plug socket, and an energy output source (first energy output source) 32. The energy output source 32 is electrically connected to the electrodes 27 and 28 via an electricity supply path (first electricity supply path) 33 which extends through the inside of the cable 10. The energy output source 32 includes a converter circuit, an amplifier circuit, etc., and converts electric power from the electric power source 31. In addition, the energy output source 32 outputs converted electric energy (high-frequency electric power). The electric energy, which is output from the energy output source 32, is supplied to the electrodes 27 and 28 through the electricity supply path 33. The output controller 26 of the processor 21 controls the driving of the energy output source 32, and controls the output of electric energy from the energy output source 32. Thereby, any one of output electric power P, output current I and output voltage V of the energy output source 32 is adjusted, and the supply of electric energy to the electrodes 27 and 28 is controlled.

In a state in which a treated target is grasped between the grasping pieces 15 and 16, electric energy is supplied from the energy output source 32 to the electrodes 27 and 28. Thereby, a high-frequency current flows between the electrodes 27 and 28 through the treated target grasped in contact with the electrodes 27 and 28. Specifically, the high-frequency current is applied to the treated target as treatment energy. By the high-frequency current flowing through the treated target, heat occurs in the treated target, and the treated target is denatured by the heat. Thereby, the treated target, such as a blood vessel, is sealed (coagulated) by using the high-frequency current. As described above, by the electric energy being supplied from the energy output source 32 to the electrodes 27 and 28 of the energy treatment instrument 2, the treatment energy (high-frequency current) is applied to the treated target grasped between the grasping pieces 15 and 16. Accordingly, in the present embodiment, the grasping pieces 15 and 16 function as an energy application section which applies the high-frequency current as treatment energy to the grasped treated target (blood vessel).

The electricity supply path 33 is provided with a current detection circuit 35 and a voltage detection circuit 36. In a state in which electric energy is being output from the energy output source 32, the current detection circuit 35 detects the output current I, and the voltage detection circuit 36 detects the output voltage V. The energy control device 3 is provided with an A/D converter 37. An analog signal relating to the current I detected by the current detection circuit 35, and an analog signal relating to the voltage V detected by the voltage detection circuit 36 are transmitted to the A/D converter 37. The A/D converter 37 converts the analog signal relating to the current I and the analog signal relating to the voltage V to digital signals, and transmits the converted digital signals to the processor 21.

In the state in which electric energy is being output from the energy output source 32, the processor 21 acquires information relating to the output current I and output voltage V of the energy output source 32. Then, based on the output current I and output voltage V, the impedance detector 23 of the processor 21 detects the impedance of the electricity supply path 33 including the grasped treated target (blood vessel) and electrodes 27 and 28. Thereby, an impedance Z between the paired grasping pieces 15 and 16 (i.e. the impedance of the grasped treated target) is detected.

As illustrated in FIG. 1, an operation button 18 functioning as an energy operation input section is attached to the housing 5. By pressing the operation button 18, an operation (signal) for outputting the electric energy from the energy output source 32 to the energy treatment instrument 2 is input to the control device 3. Incidentally, in place of the operation button 18 or in addition to the operation button 18, a footswitch or the like, which is separate from the energy treatment instrument 2, may be provided as the energy operation input section. As illustrated in FIG. 2, the processor 21 detects the presence or absence of an operation input by the energy operation input section such as the operation button 18. Based on the operation input by the operation button 18, the output controller 26 of the processor 21 controls the output of the electric energy from the energy output source 32.

Besides, in the treatment system 1 of the present embodiment, a forceps 80, which is separate from the energy treatment instrument 2, is used. A blood vessel is clamped by the forceps 80. Thereby, in a region of the blood vessel, which is grasped between the grasping pieces 15 and 16, a blood flow can be stopped.

As illustrated in FIG. 1 and FIG. 2, the treatment system 1 includes a rigid endoscope (endoscope) 60 as an observation element (observation device). The rigid endoscope 60 has a longitudinal axis C'. Here, in the rigid endoscope 60, one side of a direction along the longitudinal axis C' is a distal side (arrow C'1 side), and a side opposite to the distal side is a proximal side (arrow C'2 side). The rigid endoscope 60 includes an insertion section 61 which extends along the longitudinal axis C', and a held section 62 which is provided on the proximal side of the insertion section 61 and can be held. In addition, the treatment system 1 includes an image processing device 65 and a display device 67 such as a monitor. One end of a universal cord 66 is connected to the held section 62 of the rigid endoscope 60. The other end of the universal cord 66 is detachably connected to the image processing device 65. The image processing device 65 is electrically connected to the display device 67.

An imaging element 71 such as a CCD is provided in a distal portion of the insertion section 61 of the rigid endoscope 60. A subject is imaged by the imaging element 71. For example, in the state in which the blood vessel (treated target) is grasped between the grasping pieces 15 and 16, the grasped blood vessel, the grasping pieces 15 and 16 (end effector 7), etc. are photographed as a subject by the imaging element 71. At this time, the imaging element 71 performs, for example, stereoscopic photography. In addition, the imaging of the subject by the imaging element 71 is continuously performed with the passing of time. In the manner as described above, the grasped blood vessel is observed by using the rigid endoscope 60.

The image processing device 65 includes a processor (image processing section) 72 which executes an image process, etc., and a storage medium 73. The processor 72 is formed of an integrated circuit including a CPU, ASIC, FPGA or the like. The processor 72 may be formed of a single integrated circuit, or may be formed of a plurality of integrated circuits. The process in the processor 72 is executed according to a program stored in the processor 72 or storage medium 73. In addition, the storage medium 73 stores a processing program which is used in the processor 72, and parameters, tables, etc. which are used in arithmetic operations in the processor 72. The processor 72 can communicate with (can exchange information with) the processor 21 of the control device 3 by wire or wirelessly.

If the imaging of the subject is executed by the imaging element 71, an imaging signal is transmitted to the processor 72. Thereby, the processor 72 generates an observation image of the subject such as the grasped blood vessel. At this time, if stereoscopic photography is executed by the imaging element 71, the processor 72 generates a stereoscopic image as the observation image of the subject. In addition, since the subject is continuously imaged with the passing of time, the processor 72 generates the observation image continuously with time. The observation image generated by the processor 72 is displayed on the display device 67.

In addition, by executing image processing, the processor 72 acquires information relating to the subject from the generated observation image. For example, the processor 72 specifies the position of the grasping pieces 15 and 16 in the observation image. At this time, for example, a marker (not shown) is attached to the end effector 7, and the position of the grasping pieces 15 and 16 is specified from the position of the marker in the observation image. Besides, the position of the grasping pieces 15 and 16 may be specified based on the luminance, color or the like of the pixels which constitute the observation image. Then, in the observation image, the position where the blood vessel is grasped by the grasping pieces 15 and 16 (the grasping position of the blood vessel) is specified. In addition, the processor 72 detects, in the observation image, the forceps 80 which is separate from the energy treatment instrument 2, by setting as a detection range a predetermined range centering on the position where the blood vessel is grasped (the grasping position of the blood vessel). Here, the predetermined range is, for example, a range of a predetermined distance Lth or less from the grasping position of the blood vessel. In this case, the predetermined distance Lth is stored in the storage medium 73 or the like. In the process of detecting the forceps 80, for example, a marker (not shown) is attached to the forceps 80, and the forceps 80 is detected (extracted) based on the marker. Besides, the forceps 80 may be detected based on the color, luminance or the like of the pixels of the observation image. The image data of the observation image generated by the processor 72 is transmitted to the processor 21 of the control device 3. Further, the result of the image processing in the processor 72, such as the detection result of the position where the blood vessel is grasped (the grasping position of the blood vessel) and the forceps 80 in the observation image, is also transmitted to the processor 21 of the control device 3.

Based on the image data of the generated observation image and the result of the image processing in the processor 72, the judgement section 25 of the processor 21 judges whether the forceps 80 exists in the predetermined range from the position where the blood vessel is grasped (the grasping position of the blood vessel). Specifically, the judgement section 25 judges whether the part grasped between the grasping pieces 15 and 16 is near the forceps 80. In addition, based on the judgement result relating to the forceps 80, the output controller 26 of the processor 21 controls the output of electric energy from the energy output source 32. In accordance with the output state of electric energy from the energy output source 32, the actuation state of the energy treatment instrument 2 is switched between a first mode (first actuation mode) and a second mode (second actuation mode). In the present embodiment, the state of application of treatment energy (high-frequency current) from the energy application section (grasping pieces 15 and 16) to the grasped treated target is different between the first mode and the second mode.

Besides, in one example, an ultrasonic transducer 46 may be provided in the energy treatment instrument 2 (in the inside of the housing 5). In this case, a rod member is connected to the distal side of the ultrasonic transducer 46, and one of the grasping pieces 15 and 16 (e.g. the first grasping piece 15) is formed by a projecting portion of the rod member, the projection portion projecting from the sheath 6 toward the distal side. In addition, in this example, in the control device 3, an energy output source (second energy output source) 47 is provided in addition to the energy output source 32. The energy output source 47 is electrically connected to the ultrasonic transducer 46 via an electricity supply path (second electricity supply path) 48 which extends through the inside of the cable 10. Here, the energy output source 47 may be formed integral with the energy output source 32, or may be formed separate from the energy output source 32.

In this example, the energy output source 47 includes a converter circuit, an amplifier circuit, etc., and converts electric power from the electric power source 31. In addition, the energy output source 47 outputs converted electric energy (AC electric power). The electric energy that is output from the energy output source 47 is supplied to the ultrasonic transducer 46 through the electricity supply path 48. The output controller 26 of the processor 21 controls the driving of the energy output source 47, and controls the output of electric energy from the energy output source 47.

In the present example, electric energy (AC electric power) that is output from the energy output source 47 is supplied to the ultrasonic transducer 46. Thereby, ultrasonic vibration is generated in the ultrasonic transducer 46. The generated ultrasonic vibration is transmitted from the proximal side toward the distal side in the rod member (vibration transmitting member). Thereby, the rod member including one of the grasping pieces 15 and 16 (e.g. first grasping piece 15) vibrates. By the rod member vibrating in the state in which the treated target is grasped between the grasping pieces 15 and 16, the ultrasonic vibration is applied to the treated target as treatment energy. At this time, frictional heat is generated by the vibration, and the treated target such as the blood vessel can be cut and opened, while being sealed (coagulated), by the frictional heat.

In another example, a heater (not shown) in place of the ultrasonic transducer 46 may be provided in the end effector 7 (at least one of the grasping pieces 15 and 16). In this case, electric energy (DC electric power or AC electric power), which is output from the energy output source (47), is supplied to the heater through the electricity supply path (48). Thereby, heat is generated by the heater, and the treated target such as the blood vessel can be cut and opened, while being sealed (coagulated), by the heat generated by the heater. Also when each of the ultrasonic vibration, the heat of the heater, etc. is applied as treatment energy to the grasped treated target (blood vessel), at least one of the grasping pieces 15 and 16 functions as the energy application section which applies treatment energy to the treated target.

Next, the function and advantageous effects of the present embodiment will be described. When treatment is performed by using the treatment system 1, a surgeon holds the housing 5 of the energy treatment instrument 2, and inserts the end effector 7 into a body cavity such as an abdominal cavity. Then, a blood vessel (treated target) is disposed between the grasping pieces 15 and 16, and the grasping pieces 15 and 16 are closed relative to each other by closing the handle 12 relative to the grip 11. Thereby, the blood vessel is grasped between the grasping pieces 15 and 16. At this time, the insertion section 61 of the rigid endoscope 60 is also inserted into the body cavity, and the imaging element 71 continuously images, with the passing of time, the grasped blood vessel and grasping pieces 15 and 16 as a subject. Thereby, the grasped blood vessel is observed. In addition, for example, a high-frequency current is applied as treatment energy to the blood vessel, and a seal treatment of the grasped blood vessel is performed.

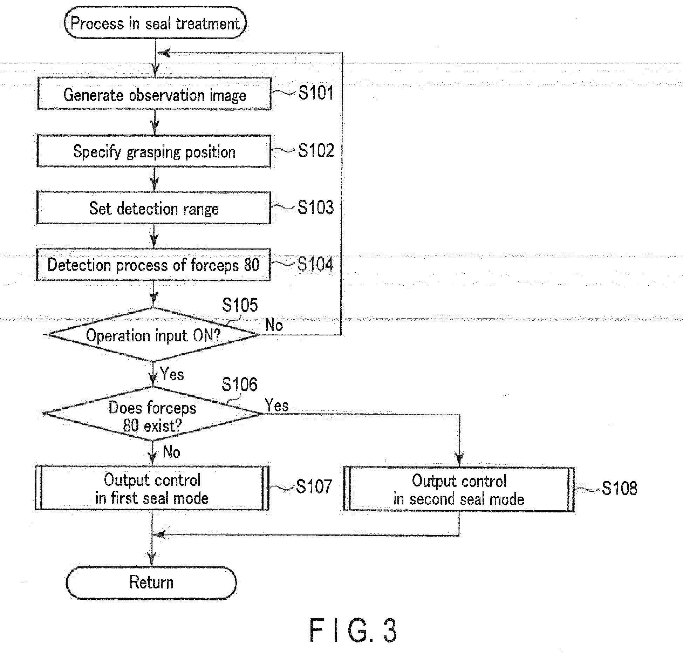

FIG. 3 is a flowchart illustrating a process in the processor 21, 72 in a seal treatment of a blood vessel using the treatment system 1 of the present embodiment. As illustrated in FIG. 3, when the seal treatment of the blood vessel is performed, the processor 72 generates an observation image, based on a subject image captured by the imaging element 71 (step S101). Then, based on the position of the marker attached to the end effector 7, the processor 72 specifies the position of the grasping pieces 15 and 16. Besides, based on the luminance of pixels, etc., the processor 72 may specify the position of the grasping pieces 15 and 16. In addition, the processor 72 specifies the position where the blood vessel is grasped (the grasping position of the blood vessel) in the observation image (step S102). At this time, the display screen of the display device 67 may be a touch panel, and the surgeon or the like may input, by the touch panel of the display device 67, an operation of indicating the position where the blood vessel is grasped in the observation image. In this case, based on the operation input on the touch panel, the processor 72 specifies the position where the blood vessel is grasped (the grasping position of the blood vessel) in the observation image. Then, the processor 72 sets the detection range of detection of the forceps 80 in the observation image to a predetermined range centering on the position where the blood vessel is grasped (the grasping position of the blood vessel) (step S103). At this time, for example, a range of a predetermined distance Lth or less from the grasping position of the blood vessel is set as the detection range. Subsequently, the processor 72 executes a detection process of the forceps 80 in the set detection range (step S104). Incidentally, as described above, the detection process of the forceps 80 is executed, for example, based on the marker attached to the forceps 80.

Then, the processor 21 of the control device 3 judges whether an operation input by the operation button (energy operation input section) 18 was executed or not (i.e. whether the operation input is ON or OFF) (step S105) . If the operation input is not executed (step S105-No), the process returns to step S101, and the processes from step S101 will be successively executed. Thus, the generation of the observation image and the detection process of the forceps 80 in the set detection range are repeatedly executed. If the operation input is executed (step S105-Yes), the judgement section 25 of the processor 21 judges whether the forceps 80 exists in the predetermined range from the position where the blood vessel is grasped (the grasping position of the blood vessel), based on the detection result in the detection process of the forceps 80 (step S106) . At this time, the judgement is made based on the observation image and the detection result in the detection process of the forceps 80 at a time point when the operation input was switched from OFF to ON, or in the neighborhood of this time point.

If it is judged that the forceps 80 does not exist in the predetermined range (step S106-No), the output controller 26 of the processor 21 executes the output control of the electric energy from the energy output source 32 in a first seal mode (step S107). If it is judged that the forceps 80 (the region where the blood vessel is clamped by the forceps 80) exists in the predetermined range from the position where the blood vessel is grasped (step S106-Yes), the output controller 26 executes the output control of the electric energy from the energy output source 32 in a second seal mode which is different from the first seal mode (step S108). Also in the state in which the operation input is executed by the operation button (energy operation input section) 18 and the treatment energy is being applied to the grasped blood vessel, the processor 72 generates the observation image, based on the subject image captured by the imaging element 71.

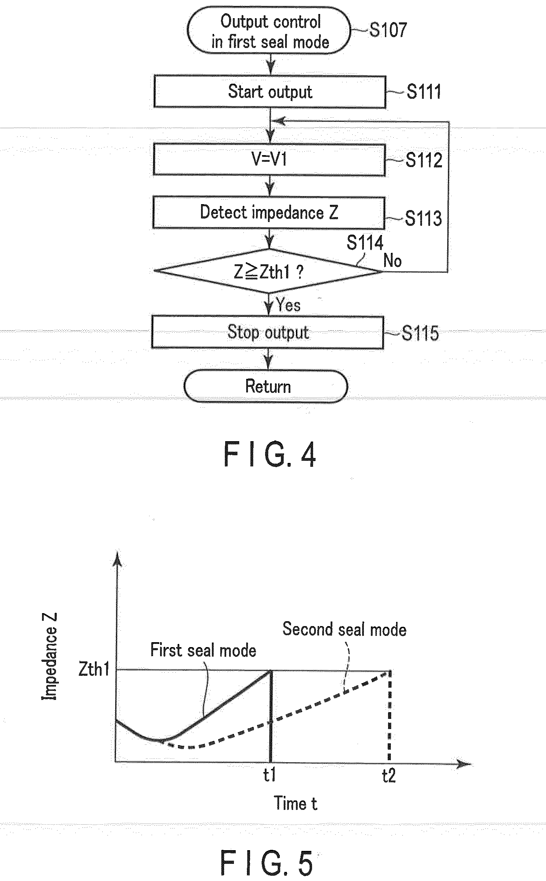

FIG. 4 is a flowchart illustrating the process of the processor 21 in the output control in the first seal mode. As illustrated in FIG. 4, in the output control in the first seal mode, the processor 21 starts the output of electric energy (high-frequency electric power) from the energy output source (first energy output source) 32 (step Sill). Thereby, the electric energy is supplied to the electrodes 27 and 28, a high-frequency current flows through the grasped blood vessel, and the blood vessel is sealed.

If a certain period of time has passed since the output start of the electric energy from the energy output source 32, the output controller 26 executes a constant voltage control for keeping constant the output voltage V from the energy output source 32 at a first voltage value V1 with the passing of time (step S112). In addition, if the output of the electric energy from the energy output source 32 is started, the impedance detector 23 of the processor 21 detects the impedance Z between the paired grasping pieces 15 and 16 (i.e. the impedance of the grasped treated target), based on the detection result of the output current I by the current detection circuit 35 and the detection result of the output voltage V by the voltage detection circuitry 36 (step S113). Then, the processor 21 judges whether the detected impedance Z is an impedance threshold (first impedance threshold) Zth1 or more (step S114). The impedance threshold Zth1 may be set by the surgeon or the like, or may be stored in the storage medium 22.

If the impedance Z is less than the impedance threshold Zth1 (step S114-No), the process returns to step S112, and the processes from step S112 will be successively executed. If the impedance Z is the impedance threshold Zth1 or more (step S114-Yes), the output controller 26 stops the output of the electric energy (high-frequency electric power) from the energy output source 32 (step S115). Thereby, the supply of electric energy to the electrodes 27 and 28 is stopped. The processor 21 executes the output control of the electric energy from the energy output source 32 in the first seal mode, and thereby the energy treatment instrument 2 is actuated in the first mode in which the grasped treated target is coagulated (the blood vessel is sealed). In the case of coagulating the treated target when the forceps 80 does not exist in the predetermined range from the position where the treated target is grasped, the energy treatment instrument 2 is actuated in the first mode.

Also in the output control in the second seal mode, like the output control in the first seal mode, the processor 21 executes the processes of step 111 and S113 to S115. However, in the second seal mode, if a certain period of time has passed since the output start of the electric energy from the energy output source 32, the output controller 26 executes a constant voltage control for keeping constant with time the output voltage V from the energy output source 32 at a second voltage value V2 which is lower than the first voltage V1. In the second seal mode, the constant voltage control is executed at the second voltage value V2 which is lower than the first voltage V1. Thus, the electric energy that is output from the energy output source 32 is lower in the second seal mode than in the first seal mode. Specifically, the output controller 26 of the processor 21 decreases the electric energy, which is output from the energy output source 32, in the second seal mode, compared to the first seal mode. The processor 21 executes the output control of the electric energy from the energy output source 32 in the second seal mode, and thereby the energy treatment instrument 2 coagulates the grasped treated target (seals the blood vessel), and is actuated in the second mode which is different from the first mode. In the case of coagulating the treated target when the forceps 80 exists in the predetermined range from the position where the treated target is grasped, the energy treatment instrument 2 is actuated in the second mode. As described above, in the present embodiment, the processor 21 controls the output of electric energy from the energy output source 32, based on the judgement result of the forceps 80. Thereby, the processor 21 switches the actuation state of the energy treatment instrument 2 between the first mode (first actuation mode) and second mode (second actuation mode). The output state of electric energy from the energy output source 32 is different between the first seal mode and second seal mode. Thus, in the energy treatment instrument 2, the application state of treatment energy (high-frequency current) from the energy application section (grasping pieces 15 and 16) to the grasped treated target is different between the first mode and the second mode.

Besides, if the electric energy, which is output from the energy output source 32, is smaller in the second seal mode than in the first seal mode, output control other than the constant voltage control may be executed in each of the first seal mode and second seal mode. For instance, in one example, in the first seal mode, the output controller 26 executes a constant electric power control for keeping constant with time the output electric power P from the energy output source 32 at a first electric power P1. In addition, in the second seal mode, the output controller 26 executes a constant electric power control for keeping constant with time the output electric power P from the energy output source 32 at a second electric power P2 which is lower than the first electric power P1. In another example, in the first seal mode, it is possible to execute both the constant voltage control for keeping constant with time the output voltage V at the first voltage value VI, and the constant electric power control for keeping constant with time the output electric power P at the first electric power P1. The constant voltage control and the constant electric power control are switched in accordance with the impedance Z. In addition, in the second seal mode, it is possible to execute both the constant voltage control for keeping constant with time the output voltage V at the second voltage value V2 that is lower than the first voltage value V1, and the constant electric power control for keeping constant with time the output electric power P at the second electric power P2 that is lower than the first electric power P1, and the constant voltage control and the constant electric power control are switched in accordance with the impedance Z. It should be noted, however, that in each example, the electric energy that is output from the energy output source 32 is smaller in the second seal mode than in the first seal mode.

Further, in the present embodiment, in each of the first seal mode and the second seal mode, only the high-frequency current is applied as treatment energy to the blood vessel, and no treatment energy other than the high-frequency current, such as ultrasonic vibration and the heat of a heater, is applied to the blood vessel (treated target). For instance, in an example in which the ultrasonic transducer 46 is provided in the energy treatment instrument 2, the processor 21 stops the output of electric energy from the energy output source 47 to the ultrasonic transducer 46 in each of the first seal mode and second seal mode. Thus, in each of the first seal mode and second seal mode, no electric energy is supplied to the ultrasonic transducer 46, nor is ultrasonic vibration generated by the ultrasonic transducer 46. Similarly, in an example in which a heater is provided in the energy treatment instrument 2, the processor 21 stops the output of electric energy from the energy output source to the heater in each of the first seal mode and second seal mode. Thus, in each of the first seal mode and second seal mode, no electric energy is supplied to the heater, nor is heat generated by the heater.

In one example, if the output control in the first seal mode or the output control in the second seal mode is finished, a transition occurs to the state in which electric energy is supplied to none of the electrodes 27 and 28, ultrasonic transducer 46 and heater, and no treatment energy, such as high-frequency current, ultrasonic vibration and the heat of the heater, is applied to the treated target. In another example, if the output control in the first seal mode or the output control in the second seal mode is finished, a transition automatically occurs to the output control in a cut-and-open mode. In this case, in the example in which the ultrasonic transducer 46 is provided in the energy treatment instrument 2, the processor 21 causes, in the cut-and-open mode, the energy output source 47 to output electric energy to the ultrasonic transducer 46 at a cut-and-open level (high output level). Thereby, ultrasonic vibration occurs in the ultrasonic transducer 46, and the ultrasonic vibration is transmitted to one of the grasping pieces 15 and 16. Then, the transmitted ultrasonic vibration is applied as treatment energy to the grasped blood vessel (treated target), and the blood vessel is cut and opened by frictional heat due to the ultrasonic vibration. Similarly, in the example in which the heater is provided in the energy treatment instrument 2, the processor 21 causes, in the cut-and-open mode, the energy output source to output electric energy to the heater at the cut-and-open level (high output level). Thereby, heat is generated by the heater. In addition, the heat of the heater is applied as treatment energy to the grasped blood vessel, and the blood vessel is cut and opened.

FIG. 5 is a view illustrating an example of a variation with time of the impedance Z between the paired grasping pieces 15 and 16 (i.e. the impedance of the grasped treated target) in the state in which the processor 21 is executing the output control in each of the first seal mode and second seal mode. In FIG. 5, the ordinate axis indicates the impedance Z, and the abscissa axis indicates time t with reference to the start of output of electric energy from the energy output source 32. Further, in FIG. 5, a solid line indicates a variation with time of the impedance Z in the first seal mode, and a broken line indicates a variation with time of the impedance Z in the second seal mode. As shown in FIG. 5, if the output of electric energy from the energy output source 32 is started and high-frequency current begins to flow through the blood vessel (treated target), such a behavior is normally exhibited that the impedance Z decreases, for a moment, with the passing of time. Then, if the impedance Z decreases with time to a certain degree, such a behavior is normally exhibited that the impedance Z increases with time in accordance with the rise in temperature of the treated target by the heat due to the high-frequency current.

In the present embodiment, as described above, the electric energy that is output from the energy output source 32 is lower in the second seal mode than in the first seal mode. Thus, compared to the first seal mode, in the second seal mode, the amount of heat per unit time generated due to the high-frequency current flowing in the blood vessel (treated target) is small. Accordingly, compared to the first seal mode, in the second seal mode, the rate of temperature rise of the treated target (blood vessel) is low, and the rate of increase of the impedance Z in the state in which the impedance Z increases with time is low. Thus, the time from the output start of electric energy from the energy output source 32 to the reaching of the impedance Z to the impedance threshold Zth1 is longer in the second seal mode than in the first seal mode. In fact, in the example of FIG. 5, in the first seal mode, the impedance Z reaches the impedance threshold Zth1 at time t1. On the other hand, in the second seal mode, the impedance Z reaches the impedance threshold Zth1 at time t2 which is later than time t1. In the present embodiment, as described above, in each of the first seal mode and second seal mode, the output of electric energy from the energy output source 32 is stopped based on the fact that the impedance Z has increased to the impedance threshold Zth1 or more. Accordingly, the output time of electric energy from the energy output source 32 is longer in the second seal mode than in the first seal mode.

As described above, compared to the first seal mode, in the second seal mode, the output controller 26 (processor 21) decreases the electric energy which is output from the energy output source 32, and increases the output time of the electric energy from the energy output source 32. Thus, compared to the first seal mode, in the second seal mode, the amount of heat per unit time generated due to the high-frequency current in the blood vessel is small, and the time of application of the high-frequency current to the blood vessel is long. Specifically, in the energy treatment instrument 2, the application time of treatment energy (high-frequency current) from the energy application section (grasping pieces 15 and 16) to the treated target (blood vessel) is longer in the second mode (second actuation mode) than in the first mode (first actuation mode). The total amount of treatment energy (high-frequency current), which is applied to the treated target in the first seal mode, corresponds to, for example, the area between the impedance Z and time t indicated by the solid line in FIG. 5. In addition, the total amount of treatment energy (high-frequency current), which is applied to the treated target in the second seal mode, corresponds to, for example, the area between the impedance Z and time t indicated by the broken line in FIG. 5. Here, in FIG. 5, the area on the lower side of the impedance Z in the second seal mode indicated by the broken line is larger than the area on the lower side of the impedance Z in the first seal mode indicated by the solid line. Accordingly, the sealing performance of the blood vessel by the high-frequency current is higher in the second seal mode than in the first seal mode.

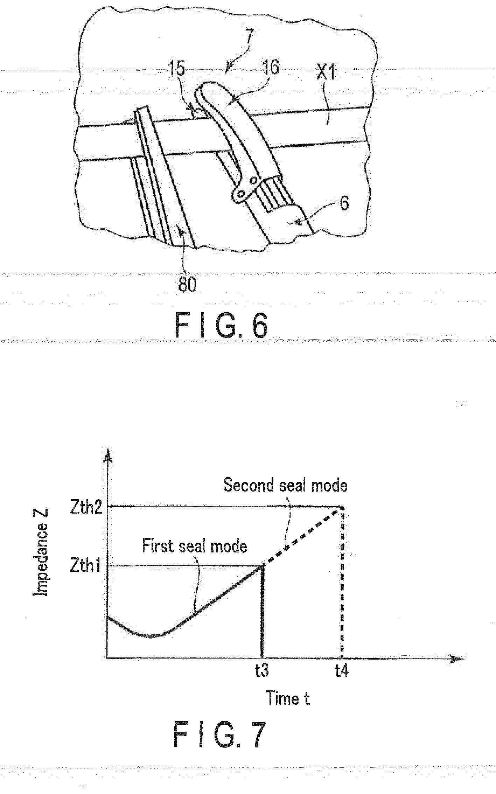

FIG. 6 is a view illustrating an example of an observation image generated by the processor 72 in a state in which a blood vessel X1 is grasped between the grasping pieces 15 and 16. As illustrated in FIG. 6, when the blood vessel X1 is grasped, there is a case in which the blood vessel X1 is clamped by the forceps 80 and the blood flow is stopped in that region of the blood vessel X1, which is grasped between the grasping pieces 15 and 16. In this case, such a situation may occur that the blood vessel X1 is grasped between the grasping pieces 15 and 16 near the region clamped by the forceps 80. Here, if the vicinity of the region clamped by the forceps 80 is treated in the same manner as when a region apart from the forceps 80 is sealed, there is possibility that the treatment of sealing the blood vessel X1 by using treatment energy such as high-frequency current is affected. Thus, there is a possibility that the sealing performance of the blood vessel X1, such as a pressure resistance value of the sealed blood vessel X1, is affected.

In the present embodiment, the processor 21 judges whether the forceps 80 exists in the predetermined range (the range of the predetermined distance Lth or less) from the position where the blood vessel is grasped (the grasping position of the blood vessel) in the observation image. Then, if the processor 21 judges that the forceps 80 does not exist in the predetermined range, the output control is executed in the first seal mode. If the processor 21 judges that the forceps 80 exists in the predetermined range, the output control is executed in the second seal mode. Thus, compared to the case in which it is judged that the forceps 80 does not exist, when it is judged that the forceps 80 exists, the electric energy that is output from the energy output source 32 is small and the output time of electric energy from the energy output source 32 is long. Specifically, in the energy treatment instrument 2, the application time of treatment energy (high-frequency current) from the energy application section (grasping pieces 15 and 16) to the treated target (blood vessel) is longer in the second mode (second actuation mode) in the case of the judgement that the forceps 80 exists in the predetermined range from the position where the treated target is grasped, than in the first mode (first actuation mode) in the case of the judgement that the forceps 80 does not exist in the predetermined range. Accordingly, when the blood vessel is grasped near the region clamped by the forceps 80, the treatment is performed in the second seal mode in which the sealing performance of the blood vessel by the high-frequency current of the energy treatment instrument 2 of the treatment system 1 is higher than in the first seal mode. Thus, the blood vessel is sealed at substantially the same level as in the case in which the blood vessel is grasped in a region apart from the region clamped by the forceps 80. Accordingly, by using the energy treatment instrument 2 of the treatment system 1, the sealing performance of the blood vessel, such as a pressure resistance value of the sealed blood vessel (difficulty in blood flow to the sealed region), is easily maintained even when the blood vessel is grasped near the region clamped by the forceps 80.

As described above, in the present embodiment, even when the forceps 80 (the region clamped by the forceps 80) exists in the predetermined range from the position where the blood vessel is grasped (the grasping position of the blood vessel), the grasped blood vessel is properly sealed by enhancing the sealing performance of the blood vessel by the high-frequency current. Specifically, even when the blood vessel is grasped near the forceps 80, the blood vessel X1 is properly sealed by using the treatment energy such as high-frequency current, and a proper treatment performance (sealing performance) is exhibited.

### (Modifications of the First Embodiment)

In a first modification of the first embodiment, the process of the processor 21 in the output control in the second seal mode is different from the first embodiment. In this modification, too, in the output control in the first seal mode, the processor 21 executes the same process as in the first embodiment (see FIG. 4). In the output control in the second seal mode, too, the processor 21 executes the process of step Sill to S113, like the output control in the first seal mode. However, in the second seal mode, instead of the process of step S114, the processor 21 judges whether the detected impedance Z is an impedance threshold (second impedance threshold) Zth2 or more. Here, the impedance threshold Zth2 is greater than the impedance threshold (first impedance threshold) Zth1. Besides, the impedance threshold Zth2 may be set by the surgeon or the like, or may be stored in the storage medium 22.

In addition, when the impedance Z is less than the impedance threshold Zth2, the process returns to S112, and the processes from step S112 will be successively executed. When the impedance Z is the impedance threshold Zth2 or more, the output controller 26 stops the output of the electric energy (high-frequency electric power) from the energy output source 32. Accordingly, in the second seal mode of the present modification, the output of electric energy from the energy output source 32 is stopped based on the fact that the impedance Z has increased to the impedance threshold (second impedance threshold) Zth2 or more, which is greater than the impedance threshold (first impedance threshold) Zth1. In this modification, too, the processor 21 controls the output of electric energy from the energy output source 32, based on the judgement result of the forceps 80. Thereby, the processor 21 switches the actuation state of the energy treatment instrument 2 between the first mode (first actuation mode) and second mode (second actuation mode). Besides, in this modification, too, the output state of electric energy from the energy output source 32 is different between the first seal mode and second seal mode. Thus, in the energy treatment instrument 2, the application state of treatment energy (high-frequency current) from the energy application section (grasping pieces 15 and 16) to the grasped treated target is different between the first mode and the second mode.

FIG. 7 is a view illustrating an example of a variation with time of the impedance Z between the paired grasping pieces 15 and 16 in the state in which the processor 21 of this modification is executing the output control in each of the first seal mode and second seal mode. In FIG. 7, the ordinate axis indicates the impedance Z, and the abscissa axis indicates time t with reference to the start of output of electric energy from the energy output source 32. Further, in FIG. 7, a solid line indicates a variation with time of the impedance Z in the first seal mode, and a broken line indicates a variation with time of the impedance Z in the second seal mode.

As described above, in the present modification, in the first seal mode, the output of electric energy from the energy output source 32 is stopped based on the fact that the impedance Z has increased to the impedance threshold Zth1 or more. On the other hand, in the second seal mode, the output of electric energy from the energy output source 32 is stopped based on the fact that the impedance Z has increased to the impedance threshold Zth2 or more. In addition, the impedance threshold Zth2 is greater than the impedance threshold Zth1. Thus, the output time of electric energy from the energy output source 32 is longer in the second seal mode than in the first seal mode. In fact, in the example of FIG. 7, in the first seal mode, the output of electric energy is stopped at time t3. On the other hand, in the second seal mode, the output of electric energy is stopped at time t4 which is later than time t3.

As described above, in the present modification, compared to the first seal mode, in the second seal mode, the output controller 26 (processor 21) increases the impedance threshold (Zth1; Zth2) which is the reference for stopping the output, and increases the output time of electric energy from the energy output source 32. Specifically, compared to the first mode (first actuation mode) in the case in which it was judged that the forceps 80 does not exist in the predetermined range from the position where the treated target is grasped, in the second mode (second actuation mode) in the case in which it was judged that the forceps 80 exists in the predetermined range, the application time of treatment energy (high-frequency current) from the energy application section (grasping pieces 15 and 16) to the treated target (blood vessel) is long. Thus, compared to the first seal mode, in the second seal mode, the time, during which the high-frequency current is applied to the blood vessel, is long, and the total amount of treatment energy (high-frequency current), which is applied to the blood vessel, is large, and therefore the sealing performance of the blood vessel by the high-frequency is enhanced. Accordingly, in this modification, too, when the blood vessel is grasped near the region clamped by the forceps 80, the treatment is performed in the second seal mode in which the sealing performance of the blood vessel by the high-frequency current in the energy treatment instrument 2 of the treatment system 1 is higher than in the first seal mode. Thus, the blood vessel is sealed at substantially the same level as in the case in which the blood vessel is grasped in a region apart from the region clamped by the forceps 80. Accordingly, by using the energy treatment instrument 2 of the treatment system 1, the sealing performance of the blood vessel, such as a pressure resistance value of the sealed blood vessel (difficulty in blood flow to the sealed region), is easily maintained even when the blood vessel is grasped near the forceps 80.

Besides, in one modification, the first embodiment and the first modification may be combined. In this case, compared to the first seal mode, in the second seal mode, the processor 21 decreases the electric energy which is output from the energy output source 32, and increases the impedance threshold (Zth1; Zth2) that is the reference for stopping the output. In this modification, too, the output state of electric energy from the energy output source 32 is different between the first seal mode and second seal mode. Thus, in the energy treatment instrument 2, the application state of treatment energy (high-frequency current) from the energy application section (grasping pieces 15 and 16) to the grasped treated target is different between the first mode and the second mode.

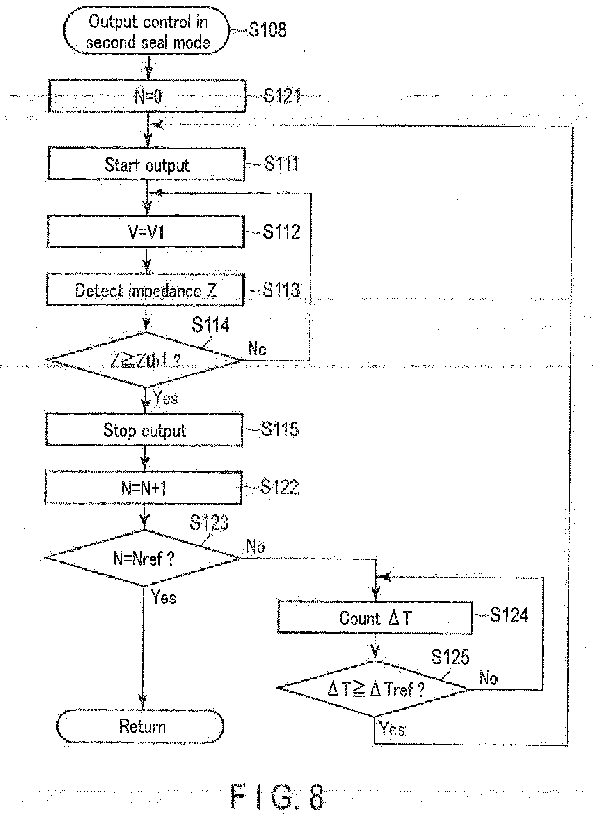

Additionally, in a second modification of the first embodiment, in the output control in the second seal mode, the processor 21 executes a process illustrated in FIG. 8. In this modification, too, in the output control in the first seal mode, the processor 21 executes the same process as in the first embodiment (see FIG. 4). In the present modification, in the output control in the second seal mode, an output number of times N of electric energy from the energy output source 32 is defined as a parameter. In the output control in the second seal mode, the processor 21 sets 0 as a default of the output number of times N (step S121). Then, like the output control in the first seal mode, the processor 21 executes the process of step S111 to S115.

If the output of electric energy from the energy output source 32 is stopped by the process in step S115, the processor 21 increments the output number of times N by 1 (step S122). Then, the processor 21 judges whether the incremented output number of times N is equal to a reference number of times Nref (step S123). The reference number of times Nref is a natural number of 2 or more, and this reference number of times Nref may be set by the surgeon or the like, or may be stored in the storage medium 22. If the output number of times N is equal to the reference number of times Nref, that is, if the output number of times N has reached the reference number of times Nref (step S123-Yes), the processor 21 finishes the output control in the second seal mode. Thereby, for example, the state in which the output of electric energy from the energy output source 32 is stopped is continuously maintained.

Here, a time (elapsed time) ΔT is defined. The time ΔT is 0 at a latest time point of time points at which the output of electric energy from the energy output source 32 was stopped by the process in step S115. If the output number of times N is not equal to the reference number of times Nref, that is, if the output number of times N has not reached the reference number of times Nref (step S123-No), the processor 21 counts the time ΔT (step S124). Then, the processor 21 judges whether the counted time ΔT is a reference time ΔTref or more (step S125). The reference time ΔTref is, for example, 10 msec, and this reference time ΔTref may be set by the surgeon or the like, or may be stored in the storage medium 22.

If the time ΔT is shorter than the reference time ΔTref (step S125-No), the process returns to step S124, and the processes from step S124 will be successively executed. Specifically, the state in which the output of electric energy from the energy output source 32 is stopped is continuously maintained, and the time ΔT is continuously counted. If the time ΔT is the reference time ΔTref or more (step S125-Yes), the process returns to step Sill, and the processes from step S111 will be successively executed. Specifically, the electric energy from the energy output source 32 is output once again.

The above process is executed. Thus, in the output control in the second seal mode, after starting the output of electric energy from the energy output source 32, the output controller 26 of the processor 21 stops the output of the electric energy. Then, after once stopping the output of electric energy from the energy output source 32, the output controller 26 resumes the output of electric energy. Specifically, in the second seal mode, if the reference time ΔTref has passed since the time point when the output of electric energy from the energy output source 32 was once stopped, the electric energy is output once again from the energy output source 32. In addition, in the output control in the second seal mode, the processor 21 causes the energy output source 32 to intermittently output electric energy by the reference number of times Nref (plural times). Also in this modification, based on the judgement result of the forceps 80, the processor 21 controls the output of electric energy from the energy output source 32, thereby switching the actuation state of the energy treatment instrument 2 between the first mode (first actuation mode) and second mode (second actuation mode). In this modification, too, the output state of electric energy from the energy output source 32 is different between the first seal mode and second seal mode. Thus, in the energy treatment instrument 2, the application state of treatment energy (high-frequency current) from the energy application section (grasping pieces 15 and 16) to the grasped treated target is different between the first mode and the second mode.

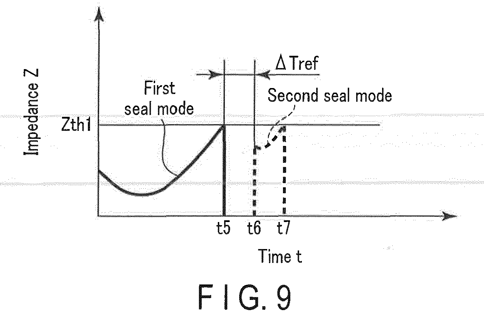

FIG. 9 is a view illustrating an example of a variation with time of the impedance Z between the paired grasping pieces 15 and 16 in the state in which the processor 21 of this modification is executing the output control in each of the first seal mode and second seal mode. In FIG. 9, the ordinate axis indicates the impedance Z, and the abscissa axis indicates time t with reference to the start of output of electric energy from the energy output source 32. Further, in FIG. 9, a solid line indicates a variation with time of the impedance Z in the first seal mode, and a broken line indicates a variation with time of the impedance Z in the second seal mode. In the example shown in FIG. 9, in each of the first seal mode and second seal mode, the output of electric energy from the energy output source 32 is stopped at time t5, based on the fact that the impedance Z has reached the impedance threshold Zth1.

As described above, in the present modification, in the second seal mode, electric energy is intermittently output from the energy output source 32 by a plurality of times (reference number of times Nref). Thus, in the example shown in FIG. 9, in the second seal mode, the output of electric energy from the energy output source 32 is resumed at time t6 when the reference time ΔTref has passed since time t5 at which the output was stopped. At this time, the impedance Z is lower than the impedance threshold Zth1. Further, at time t7 after the time t6 (at which the output of electric energy was resumed), based on the fact that the impedance Z has reached the impedance threshold Zth1, the output of electric energy from the energy output source 32 is stopped once again. Besides, in the example of FIG. 9, the reference number of times Nref is 2.

As described above, in the present modification, in the second seal mode, the output controller 26 (processor 21) resumes the output of electric energy after once stopping the output. Thereby, the output time of electric energy from the energy output source 32 becomes longer in the second seal mode than in the first seal mode, and the time of application of high-frequency current to the blood vessel becomes longer in the second seal mode than in the first seal mode. Specifically, compared to the first mode (first actuation mode) in the case in which it was judged that the forceps 80 does not exist in the predetermined range from the position where the treated target is grasped, in the second mode (second actuation mode) in the case in which it was judged that the forceps 80 exists in the predetermined range, the application time of treatment energy (high-frequency current) from the energy application section (grasping pieces 15 and 16) to the treated target (blood vessel) is long. Thus, compared to the first seal mode, in the second seal mode, the sealing performance of the blood vessel by the high-frequency is enhanced. Accordingly, in this modification, too, when the blood vessel is grasped near the region clamped by the forceps 80, the treatment is performed in the second seal mode in which the sealing performance of the blood vessel by the high-frequency current in the energy treatment instrument 2 of the treatment system 1 is higher than in the first seal mode. Thus, the blood vessel is sealed at substantially the same level as in the case in which the blood vessel is grasped in a region apart from the region clamped by the forceps 80. Accordingly, by using the energy treatment instrument 2 of the treatment system 1, the sealing performance of the blood vessel, such as a pressure resistance value of the sealed blood vessel (difficulty in blood flow to the sealed region), is easily maintained even when the blood vessel is grasped near the forceps 80.

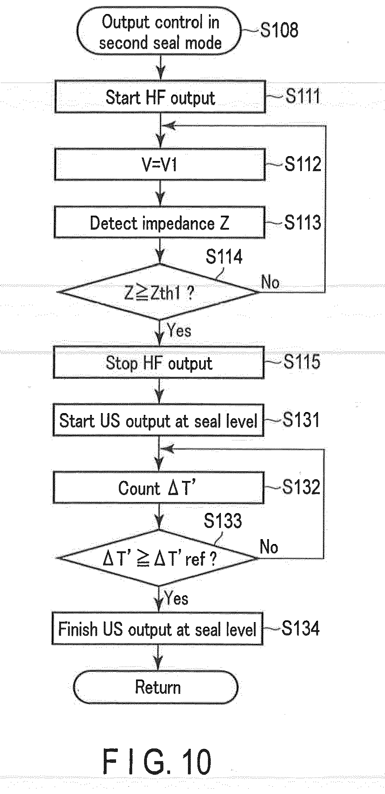

Additionally, in a third modification of the first embodiment, in the output control in the second seal mode, the processor 21 executes a process illustrated in FIG. 10. In this modification, too, in the output control in the first seal mode, the processor 21 executes the same process as in the first embodiment (see FIG. 4). In addition, also in the output control in the second seal mode, like the output control in the first seal mode, the processor 21 executes the process of steps Sill to S115.

In the second seal mode, if the output of electric energy from the energy output source 32 is stopped by the process in step S115, the output controller 26 of the processor 21 starts the output of electric energy from the energy output source 47 to the ultrasonic transducer 46 (step S131). At this time, the energy output source 47 outputs electric energy at a seal level which is a low output level. Specifically, in the output of electric energy at the seal level, the output level is lower than the output of electric energy at the above-described cut-and-open level. Thus, in the output at the seal level, compared to the output at the cut-and-open level, the electric energy supplied to the ultrasonic transducer 46 is low, and the amplitude of ultrasonic vibration transmitted to one of the grasping pieces 15 and 16 is small. Accordingly, in the output at the seal level, the amount of frictional heat by the ultrasonic vibration is small. The grasped blood vessel is not cut and opened by the frictional heat, and only sealing of the blood vessel is performed. In FIG. 10, the output of electric energy from the energy output source 32 to the electrodes 27 and 28 is indicated as "HF (high-freguency) output", and the output of electric energy from the energy output source 47 to the ultrasonic transducer 46 is indicated as "US (ultrasonic) output".

Here, a time (elapsed time) ΔT' is defined. The time ΔT' is 0 at a time point when the output of electric energy from the energy output source 47 was started at the seal level by the process in step S131 (a time point when the output from the energy output source 32 was stopped by the process in step S115). If the output of electric energy from the energy output source 47 is started at the seal level, the processor 21 counts the time ΔT' (step S132). Then, the processor 21 judges whether the counted time ΔT' is a reference time ΔT'ref or more (step S133). The reference time ΔT'ref may be set by the surgeon or the like, or may be stored in the storage medium 22.

If the time ΔT' is shorter than the reference time ΔT'ref (step S133-No), the process returns to step S132, and the processes from step S132 will be successively executed. Specifically, the time ΔT' is continuously counted. If the time ΔT' is the reference time ΔT'ref or more (step S133-Yes), the output controller 26 ends the output of electric energy from the energy output source 47 at the seal level (step S134). At this time, the output of electric energy from the energy output source 47 to the ultrasonic transducer 46 may be stopped. Alternatively, a transition may automatically occur to the output control in the cut-and-open mode, and a change may automatically be made to a state in which electric energy is output to the ultrasonic transducer 46 at the cut-and-open level (high output level). Besides, in one example, instead of the process of step S132 and S133, the output controller 26 may end the output of electric energy at the seal level from the energy output source 47, based on the fact that the operation of the operation button (energy operation input section) 18 was released (i.e. the fact that the operation input was turned OFF).

As described above, in the present modification, in the second seal mode, if the output controller 26 (processor 21) stops the output of electric energy to the electrodes 27 and 28, the output controller 26 starts the output of electric energy to the ultrasonic transducer 46. Specifically, based on the judgement result of the forceps 80, the processor 21 controls the output of electric energy from the energy output source 32, 47, thereby switching the actuation state of the energy treatment instrument 2 between the first mode (first actuation mode) and the second mode (second actuation mode). In addition, in the present modification, electric energy is output from the energy output source 47 only in the second seal mode. Thus, in the energy treatment instrument 2, the state of application of treatment energy (high-frequency current and ultrasonic vibration) from the energy application section (grasping pieces 15 and 16) to the grasped treated target is different between the first mode and the second mode. Thus, in the second seal mode, even after the output of electric energy to the electrodes 27 and 28 is stopped, the grasped blood vessel is sealed by the ultrasonic vibration (frictional heat). Specifically, in the second seal mode, even in the state in which the impedance Z is high and it is difficult for high-frequency current to flow in the blood vessel, the blood vessel is sealed by the frictional heat due to ultrasonic vibration. Thus, compared to the first seal mode, in the second seal mode, the sealing performance of the blood vessel by the treatment energy is enhanced. Accordingly, in this modification, too, when the blood vessel is grasped near the region clamped by the forceps 80, the treatment is performed in the second seal mode in which the sealing performance of the blood vessel by the treatment energy in the energy treatment instrument 2 of the treatment system 1 is higher than in the first seal mode. Thus, the blood vessel is sealed at substantially the same level as in the case in which the blood vessel is grasped in a region apart from the region clamped by the forceps 80. Accordingly, by using the energy treatment instrument 2 of the treatment system 1, the sealing performance of the blood vessel, such as a pressure resistance value of the sealed blood vessel (difficulty in blood flow to the sealed region), is easily maintained even when the blood vessel is grasped near the forceps 80.

Besides, in one modification, in the second seal mode, if the output of electric energy from the energy output source 32 is stopped by the process in step S115, the output controller 26 of the processor 21 starts the output of electric energy to the heater. At this time, too, the electric energy is output at the seal level which is a lower output level than the above-described cut-and-open level. Thus, in the output at the seal level, compared to the output at the cut-and-open level, the electric energy supplied to the heater is small. Accordingly, in the output at the seal level, the amount of heat generated by the heater is small. The grasped blood vessel is not cut and opened by the heat of the heater, and only sealing of the blood vessel is performed. In this modification, in the second seal mode, the blood vessel is sealed by the heat of the heater in addition to the high-frequency current. Specifically, in the present modification, too, in the energy treatment instrument 2, the application state of treatment energy (the high-frequency current and the heat of the heater) from the energy application section (grasping pieces 15 and 16) to the grasped treated target is different between the first mode and the second mode. Accordingly, the sealing performance of the blood vessel by the treatment energy is higher in the second seal mode than in the first seal mode. Therefore, the same function and advantageous effects as in the third modification of the first embodiment can be obtained.

In the case in which it was judged that the forceps 80 exists in the predetermined range from the grasping position, the output control of electric energy is executed in which the sealing performance of the blood vessel by the treatment energy is enhanced, compared to the case in which it was judged that the forceps 80 does not exist in the predetermined range. This output control is also applicable to an example in which only the treatment energy other than the high-frequency current (e.g. the ultrasonic vibration and the heat of the heater) is applied to the blood vessel, without the high-frequency current being applied to the blood vessel. For instance, in one modification in which electric energy is output to the ultrasonic transducer 46 at the seal level and the blood vessel is sealed by using only the ultrasonic vibration, the processor 21 decreases the electric energy which is output from the energy output source 47 to the ultrasonic transducer 46, and increases the output time of electric energy to the ultrasonic transducer 46, in the second seal mode (the second mode of the energy treatment instrument 2), compared to the first seal mode (the first mode of the energy treatment instrument 2). Thereby, compared to the first seal mode (the case in which it was judged that the forceps 80 does not exist), in the second seal mode (the case in which it was judged that the forceps 80 exists), the time of application of ultrasonic vibration to the blood vessel is long, and the sealing performance of the blood vessel by the ultrasonic vibration is enhanced. In addition, in one modification in which electric energy is output to the heater at the seal level and the blood vessel is sealed by using only the heat of the heater, the processor 21 makes lower the electric energy which is output from the energy output source to the heater, and makes longer the output time of electric energy to the heater in the second seal mode than in the first seal mode. Thereby, compared to the first seal mode (the case in which it was judged that the forceps 80 does not exist), in the second seal mode (the case in which it was judged that the forceps 80 exists), the time of application of the heat of the heater to the blood vessel is increased, and the sealing performance of the blood vessel by the heat of the heater is enhanced. Accordingly, by using the energy treatment instrument 2 of the treatment system 1, the sealing performance of the blood vessel, such as a pressure resistance value of the sealed blood vessel (difficulty in blood flow to the sealed region), is easily maintained even when the blood vessel is grasped near the forceps 80.

Besides, in one modification, the surgeon or the like may judge whether the processor 21 is caused to execute the output control in the first seal mode or to execute the output control in the second seal mode. In this modification, two operation buttons or the like, which are energy operation input sections, are provided. If the operation input is executed by one of the operation buttons, the processor 21 (output controller 26) executes the output control of electric energy in the first seal mode, and the energy treatment instrument 2 is actuated in the first mode (first actuation mode) for coagulating the treated target. If the operation input is executed by the other operation button, the processor 21 executes the output control of electric energy in the second seal mode in which the sealing performance of the blood vessel by the treatment energy is higher than in the first seal mode. Thereby, the energy treatment instrument 2 is actuated in the second mode (second actuation mode) which is different from the first mode with respect to the application state of treatment energy to the treated target. Compared to the first mode, in the second mode, the coagulation performance of the treated target by the treatment energy (the sealing performance of the blood vessel by the treatment energy) is high. In the present modification, a notification section (not shown), which indicates a judgement result as to whether the forceps 80 exists in the predetermined range from the position where the blood vessel is grasped (the grasping position of the blood vessel), is provided in, for example, the control device 3. In one example, the notification section is an LED, and the LED is turned on when it was judged that the forceps 80 exists in the predetermined range. In another example, the notification section may be a buzzer, a display screen, etc.

Besides, in another modification, the display device 67 functions as a notification section, and at least one of an observation image and a result of image processing is displayed on the display device 67. In this modification, based on the observation image and/or the result of image processing, which is displayed on the display device 67, the surgeon judges whether the forceps 80 (the region where the blood vessel is clamped by the forceps 80) exists in the predetermined range from the position where the blood vessel is grasped (the grasping position of the blood vessel). Then, the surgeon judges which of the two operation buttons is to be operated to execute the operation input, and selects whether the processor 21 is caused to execute the output control in the first seal mode or to execute the output control in the second seal mode.

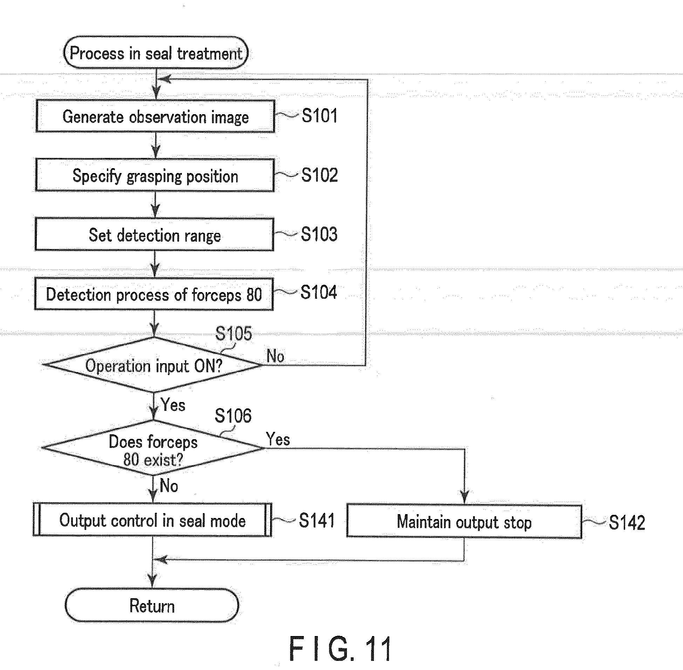

Additionally, in a fourth modification of the first embodiment, in the seal treatment of the blood vessel, the processor 21, 72 executes a process illustrated in FIG. 11. In the present modification, like the above-described embodiment, etc., in the seal treatment of the blood vessel, the processor 21, 72 executes the process of steps S101 to S106. Then, if it is judged that the forceps 80 does not exist in the predetermined range from the grasping position of the blood vessel (step S106-No), the processor 21 executes the output control of electric energy in the seal mode (step S141). In the output control in the seal mode, the processor 21 executes, for example, the same process as the output control in the first seal mode of the first embodiment (see FIG. 4). The processor 21 executes the output control of the electric energy in the seal mode, and thereby the energy treatment instrument 2 is actuated in the first mode for coagulating the grasped treated target (sealing the blood vessel). If it is judged that the forceps 80 exists in the predetermined range (step S106-Yes), the processor 21 maintains the output stop of electric energy, regardless of the presence/absence of the operation input by the operation button 18 (step S142). At this time, the energy treatment instrument 2 is actuated in the second mode. Specifically, the output of electric energy from the energy output source 32, 47 is continuously stopped. Thus, when it was judged that the forceps 80 exists, even" if the operation input is executed by the operation button 18, treatment energy such as high-frequency current is not applied to the grasped blood vessel. Accordingly, also in the present modification, based on the judgement result of the forceps 80, the processor 21 controls the output of electric energy from the energy output source 32, thereby switching the actuation state of the energy treatment instrument 2 between the first mode (first actuation mode) and second mode (second actuation mode). In this modification, in the second mode, the output of electric energy from the energy output source 32, 47 is stopped. Thus, in the energy treatment instrument 2, the application state of treatment energy (high-frequency current, etc.) from the energy application section (grasping pieces 15 and 16) to the grasped treated target is different between the first mode and the second mode.

The output control is executed as described above. Thereby, in the present modification, when the forceps 80 exists in the predetermined range from the position where the blood vessel is grasped (the grasping position of the blood vessel), no treatment energy is applied to the blood vessel. Specifically, in the state in which the sealing performance is affected, for example, in such a case that the blood vessel is grasped near the region clamped by the forceps 80, no treatment energy is applied to the blood vessel. Treatment energy is applied to the blood vessel only in the state in which the influence on the sealing performance is small, for example, in such a case that the blood vessel is grasped in a region apart from the region clamped by the forceps 80. Thus, the blood vessel is properly sealed by using the treatment energy such as high-frequency current, and a proper treatment performance (sealing performance) is exhibited.

Besides, in one modification, the surgeon or the like may judge whether or not to output electric energy in the seal mode. In the present modification, the above-described notification section is provided in, for example, the control device 3. In addition, when it was notified or judged that the forceps 80 does not exist in the predetermined range from the grasping position of the blood vessel, the surgeon executes the operation input by the operation button 18, and causes the processor 21 to execute the output control in the seal mode. Thereby, electric energy is output from the energy output source 32, 47, and the energy treatment instrument 2 is actuated in the first mode (first actuation mode). On the other hand, when it was notified or judged that the forceps 80 (the region clamped by the forceps 80) exists in the predetermined range, the surgeon does not execute the operation input by the operation button 18. Thus, no electric energy is output from the energy output source 32, 47, and the energy treatment instrument 2 is actuated in the second mode (second actuation mode) which is different from the first mode.

### (Second Embodiment)

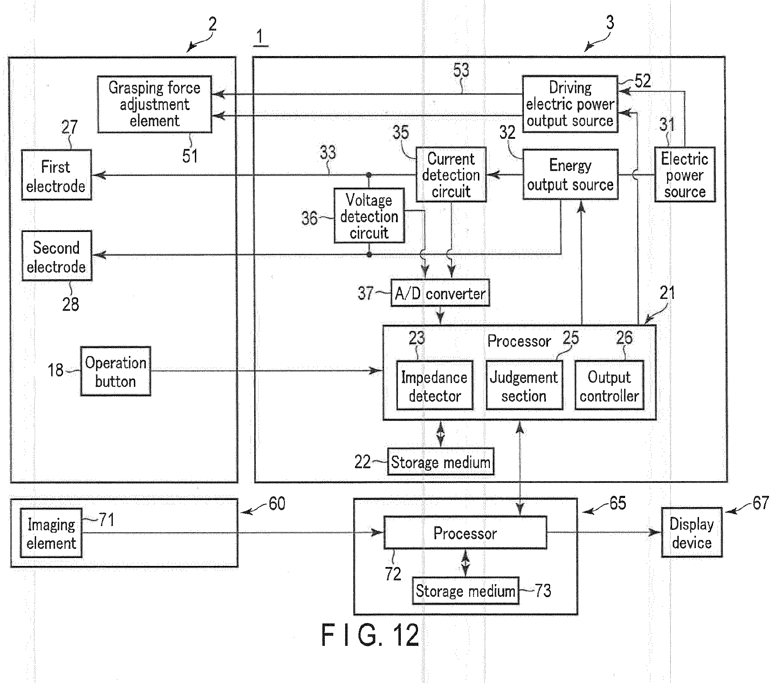

Next, a second embodiment of the present invention will be described with reference to FIG. 12 to FIG. 14. In the second embodiment, the configuration of the first embodiment is modified as described below. Incidentally, the same parts as in the first embodiment are denoted by like reference numerals, and a description thereof is omitted.

FIG. 12 is a view illustrating a control configuration in a treatment system 1 in the present embodiment. As illustrated in FIG. 12, in the present embodiment, a grasping force adjustment element 51 is provided in the energy treatment instrument 2. A grasping force of the treated target (blood vessel) between the grasping pieces 15 and 16 varies in accordance with a driving state of the grasping force adjustment element 51. Specifically, the grasping force of the treated target between the grasping pieces 15 and 16 is adjusted by the grasping force adjustment element 51. In addition, in this embodiment, a driving electric power output source 52 is provided in the control device 3. The driving electric power output source 52 is electrically connected to the grasping force adjustment element 51 via an electricity supply path 53 extending through the inside of the cable 10. Here, the driving electric power output source 52 may be formed integral with the above-described energy output source 32, 47, or may be formed separate from the energy output source 32, 47.

The driving electric power output source 52 includes a converter circuit, an amplifier circuit, etc., and converts electric power from the electric power source 31 to driving electric power to the grasping force adjustment element 51. In addition, the driving electric power output source 52 outputs the converted driving electric power, and the output driving electric power is supplied to the grasping force adjustment element 51 through the electricity supply path 53. The processor 21 controls the driving of the driving electric power output source 52, and controls the output of driving electric power from the driving electric power output source 52. Thereby, the supply of driving electric power to the grasping force adjustment element 51 is controlled, and the driving of the grasping force adjustment element 51 is controlled. In the present embodiment, in accordance with the driving state of the grasping force adjustment element 51, the actuation state of the energy treatment instrument 2 is switched between the first mode (first actuation mode) and the second mode (second actuation mode). In the present embodiment, the grasping force of the treated target (blood vessel) between the grasping pieces 15 and 16 is different between the first mode and the second mode.

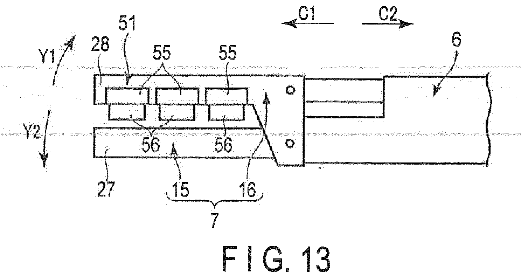

FIG. 13 is a view illustrating an example of the grasping force adjustment element 51. In the example illustrated in FIG. 13, as the grasping force adjustment element 51, a heater 55 and a volume change portion 56 are provided in the second grasping piece 16. The volume change portion 56 is formed of an electrically insulating material such as parylene, nylon or ceramics. By closing the grasping pieces 15 and 16 relative to each other, the volume change portion 56 can come in contact with the first grasping piece 15 (first electrode 27). In the state in which the volume change portion 56 is in contact with the first grasping piece 15, the electrodes 27 and 28 are spaced apart from each other, and a contact between the electrodes 27 and 28 is prevented by the volume change portion 56. In addition, the volume change portion 56 is formed of a material with a high thermal expansion coefficient.

Driving electric power is output from the driving electric power output source 52 to the heater 55. Thereby, the grasping force adjustment element 51 is driven, and heat is generated by the heater 55. By the heat generated by the heater 55, the temperature of the volume change portion 56 rises, and the volume change portion 56 expands (the volume of the volume change portion 56 increases). By the volume change portion 56 expanding in the state in which the blood vessel (treated target) is grasped between the grasping pieces 15 and 16, the distance between the grasping pieces 15 and 16 decreases, and the grasping force of the treated target between the grasping pieces 15 and 16 increases. In addition, in this example, coagulation, cutting and opening, etc. of the treated target are not performed by the heat generated by the heater 55.

Besides, in another example, a Peltier element may be provided in place of the heater 55. In this case, by the driving electric power being output to the Peltier element from the driving electric power output source 52, the Peltier element transfers heat to the volume change portion 56 side. By the transfer of heat by the Peltier element, the temperature of the volume change portion 56 rises, and the volume change portion 56 expands. Thus, as described above, in the state in which the blood vessel (treated target) is grasped between the grasping pieces 15 and 16, the distance between the grasping pieces 15 and 16 decreases, and the grasping force of the treated target between the grasping pieces 15 and 16 increases.

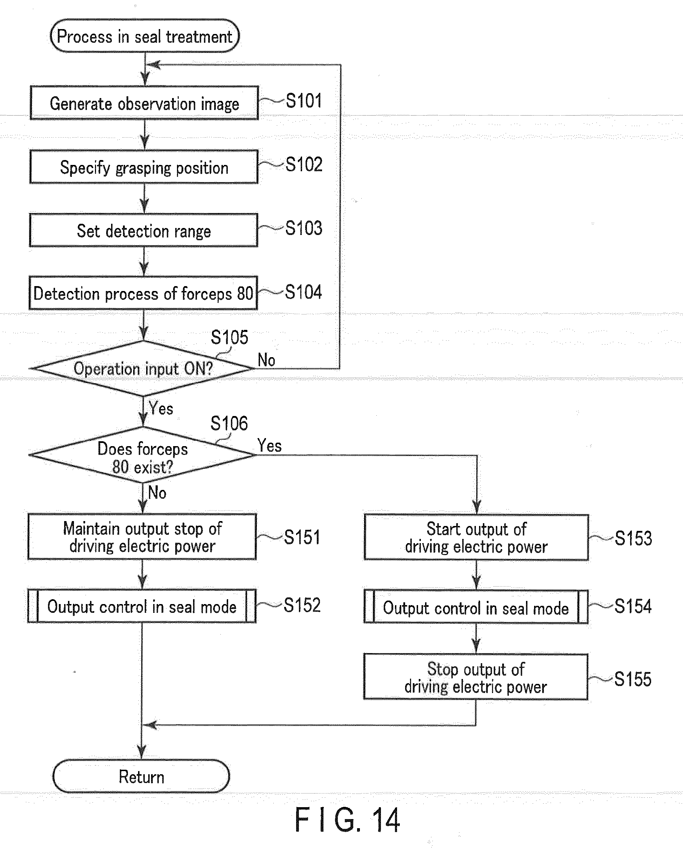

Next, the function and advantageous effects of the present embodiment will be described. FIG. 14 is a flowchart illustrating a process in the processor 21, 72 in the seal treatment of the blood vessel using the treatment system 1 of the present embodiment. In the present embodiment, like the above-described embodiment, etc., in the seal treatment of the blood vessel, the processor 21 executes the process of steps S101 to S106. Then, if it is judged that the forceps 80 does not exist in the predetermined range from the grasping position of the blood vessel (step S106-No), the processor 21 maintains the state in which the output of driving electric power from the driving electric power output source 52 to the grasping force adjustment element 51 is stopped (step S151). Thus, the grasping force adjustment element 51 is not driven, and the volume change portion 56 does not expand. Accordingly, the grasping force of the treated target between the grasping pieces 15 and 16 is maintained. In addition, the processor 21 executes the output control of electric energy from the energy output source 32 or the like in the seal mode (step S152). In the output control in the seal mode, the processor 21 executes, for example, the same process as the output control in the first seal mode of the first embodiment (see FIG. 4). In the state in which the output of driving electric power from the driving electric power output source 52 to the grasping force adjustment element 51 is stopped by the processor 21 and the grasping force adjustment element 51 is not driven, the energy treatment instrument 2 is actuated in the first mode (first actuation mode) for coagulating the grasped treated target (sealing the blood vessel).

On the other hand, if it is judged that the forceps 80 exists in the predetermined range (step S106-Yes), the processor 21 starts the output of driving electric power from the driving electric power output source 52 to the grasping force adjustment element 51 (step S153). Thus, the grasping force adjustment element 51 is driven, and the volume change portion 56 expands. Accordingly, the grasping force of the treated target between the grasping pieces 15 and 16 increases. In addition, the processor 21 executes the output control of electric energy from the energy output source 32 or the like in the seal mode (step S154). In the output control in the seal mode, the processor 21 executes, for example, the same process as the output control in the first seal mode of the first embodiment (see FIG. 4). If the output control in the seal mode is finished, the processor 21 stops the output of driving electric power from the driving electric power output source 52 to the grasping force adjustment element 51 (step S155). In the state in which the driving electric power from the driving electric power output source 52 to the grasping force adjustment element 51 is output by the processor 21 and the grasping force adjustment element 51 is driven, the energy treatment instrument 2 is actuated in the second mode (second actuation mode) which is different from the first mode and in which the grasped treated target is coagulated (the blood vessel is sealed). As described above, in the present embodiment, based on the judgement result of the forceps 80, the processor 21 controls the output of driving electric power from the driving electric power output source 52, thereby switching the actuation state of the energy treatment instrument 2 between the first mode (first actuation mode) and second mode (second actuation mode). In the energy treatment instrument 2, the driving state of the grasping force adjustment element 51 is different between the first mode and second mode. Thus, the grasping force of the treated target (blood vessel) between the grasping pieces 15 and 16 is different between the first mode and the second mode.

In the present embodiment, the control by the processor 21 is executed as described above. Thus, compared to the case in which it was judged that the forceps 80 does not exist in the predetermined range from the grasping position of the blood vessel, in the case in which it was judged that the forceps 80 exists in the predetermined range, the processor 21 increases the grasping force of the blood vessel (treated target) between the grasping pieces 15 and 16. Specifically, in the energy treatment instrument 2, the grasping force of the blood vessel (treated target) between the grasping pieces 15 and 16 is larger in the second mode (second actuation mode) than in the first mode (first actuation mode). Thus, even when the forceps 80 (the part clamped by the forceps 80) exists in the predetermined range from the position where the blood vessel is grasped (the grasping position of the blood vessel), the grasped blood vessel is properly sealed by increasing the grasping force of the blood vessel between the grasping pieces 15 and 16. Specifically, even when the blood vessel is grasped near the forceps 80, the blood vessel is properly sealed by using the treatment energy, and a proper treatment performance (sealing performance) is exhibited.

### (Modifications of the Second Embodiment)

The grasping force adjustment element 51 is not restricted to the above configuration. For example, in one modification, as the grasping force adjustment element 51, an electric motor and an abutment member are provided. In this case, by closing the handle 12 relative to the grip 11, the handle 12 comes in contact with the abutment member, and the handle 12 closes relative to the grip 11 until abutting on the abutment member. In addition, the processor 21 (output controller 26) controls the output of driving electric power from the driving electric power output source 52 to the electric motor, and controls the driving of the electric motor. By the electric motor being driven, the abutment member moves and the position of the abutment member shifts. Thereby, the stroke of the handle at a time when the handle 12 closes relative to the grip 11 changes. In the present modification, the processor 21 adjusts the position of the abutment member, based on a load σ. Thereby, compared to the case (the first mode of the energy treatment instrument 2) in which it was judged that the forceps 80 does not exist in the predetermined range from the grasping position of the blood vessel, in the case (the second mode of the energy treatment instrument 2) in which it was judged that the forceps 80 exists in the predetermined range, the processor 21 increases the stroke at the time when the handle 12 closes. Thus, in this modification, too, compared to the case in which it was judged that the forceps 80 does not exist in the predetermined range, in the case in which it was judged that the forceps 80 exists in the predetermined range, the grasping force of the blood vessel (treated target) between the grasping pieces 15 and 16 increases.

Besides, in the case of the configuration in which one of the grasping pieces 15 and 16 is formed by a rod member which is inserted through the sheath 6, a support member which supports the rod member on the most distal side within the sheath 6, and an electric motor or the like which is driven to move the support member, are provided as the grasping force adjustment element 51. In this case, by driving the electric motor or the like in accordance with the judgement result of the forceps 80, the position where the rod member is supported by the support member is changed. Thereby, in the state in which the treated target (blood vessel) is grasped between the grasping pieces 15 and 16, the amount of deflecting of the distal portion (one of grasping pieces 15 and 16) of the rod member varies, and the grasping force between the grasping pieces 15 and 16 varies. In addition, like the second embodiment, the control for adjusting the grasping force is applicable as needed, if the grasping force adjustment element 51 is provided for varying the grasping force of the treated target (blood vessel) between the grasping pieces 15 and 16.

In another modification, an operation button or the like may be provided as a driving operation input section which causes the driving electric power output source 52 to output driving electric power. In this modification, the surgeon or the like judges whether or not to output driving electric power. Besides, in this modification, the above-described notification section is provided in, for example, the control device 3 or display device 67. When it was notified or judged that the forceps 80 does not exist in the predetermined range from the grasping position of the blood vessel, the surgeon does not execute the operation input by the operation button (driving operation input section). Thus, driving electric power is not output from the driving electric power output source 52 to the grasping force adjustment element 51 (heater 55), and the volume change portion 56 does not expand. Thereby, the energy treatment instrument 2 is actuated in the first mode (first actuation mode). On the other hand, when it was notified or judged that the forceps 80 (the region clamped by the forceps 80) exists in the predetermined range, the surgeon executes the operation input by the operation button 18. Thereby, driving electric power is output from the driving electric power output source 52 to the grasping force adjustment element 51 (heater 55), and the volume change portion 56 expands by the heat generated by the heater 55. Accordingly, the energy treatment instrument 2 is actuated in the second mode (second actuation mode), and the grasping force of the treated target between the grasping pieces 15 and 16 increases.

### (Other Modifications)

In one modification, any one of the first embodiment and modifications thereof and any one of the second embodiment and modifications thereof may be combined. In this case, when it was judged that the forceps 80 does not exist in the predetermined range from the grasping position of the blood vessel, the processor 21 executes the output control of electric energy from the energy output source 32, 47 in the first seal mode, and applies treatment energy to the blood vessel. In addition, when it was judged that the forceps 80 exists in the predetermined range, the processor 21 executes the output control of electric energy from the energy output source 32, 47 in the second seal mode in which the sealing performance of the blood vessel by the treatment energy is higher than in the first seal mode, and applies treatment energy to the blood vessel. Specifically, in this modification, like the first embodiment, the sealing performance of the blood vessel by the treatment energy is higher in the second mode of the energy treatment instrument 2 than in the first mode. Further, in this modification, compared to the case (the first mode of the energy treatment instrument 2) in which it was judged that the forceps 80 does not exist in the predetermined range, in the case (the second mode of the energy treatment instrument 2) in which it was judged that the forceps 80 exists in the predetermined range, the processor 21 increases the grasping force of the treated target between the grasping pieces 15 and 16.

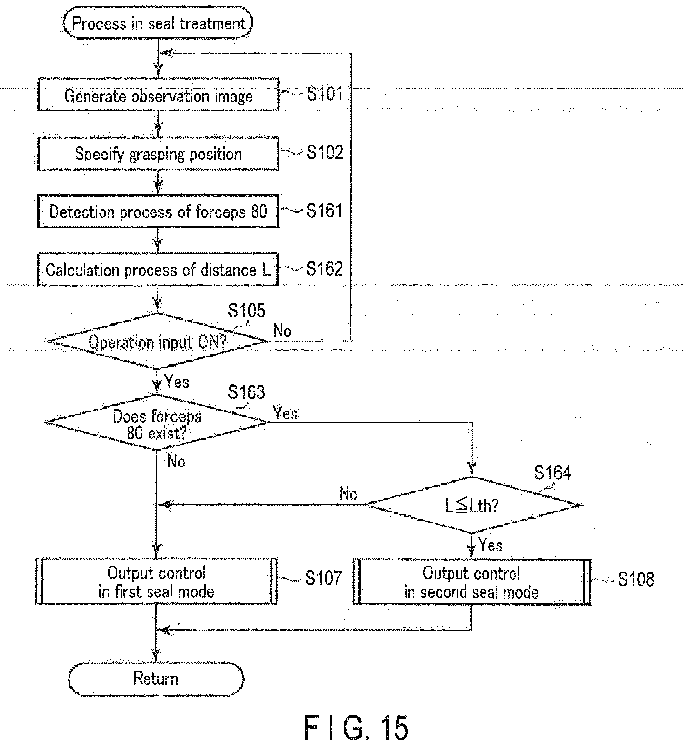

Besides, in one modification, as illustrated in FIG. 15, if the processor 72 specifies the position where the blood vessel is grasped in the observation image (step S102), the processor 72 executes as an image process a detection process of the forceps 80 by setting the entirety of the observation image as a detection range (step S161). Then, the processor 72 of the image processing device 65 specifies the position of the forceps 80 which was detected in the detection process of the forceps 80, and executes a calculation process of a distance L between the detected forceps 80 (the region clamped by the forceps 80) and the grasping position of the blood vessel (step S162). Also in this modification, as described above, the detection process of the forceps 80 is executed, for example, based on the marker attached to the forceps 80, or the luminance, color or the like of the pixels. Further, also in this modification, when the operation input is not executed (step S105-No), the process returns to step S101, and the processes from step S101 will be successively executed. Thus, the generation of the observation image and the detection process of the forceps 80 in the entirety of the observation image are repeatedly executed.

Besides, if the operation input is executed (step S105-Yes), the judgement section 25 of the processor 21 judges whether the forceps 80 exists or not, with respect to the entirety of the observation image, based on the judgement result in the detection process of the forceps 80 (step S163) . If it is judged that the forceps 80 does not exist (step S163-No), the processor executes, for example, the output control in the first seal mode (step S107). On the other hand, if it is judged that the forceps 80 exists (step S163-Yes), the judgement section 25 judges whether the calculated distance L is a predetermined distance Lth or less, based on the calculation result in the calculation process of the distance L between the detected forceps 80 and the grasping position of the blood vessel (step S164). The predetermined distance Lth is stored in, for example, the storage medium 22 or the like. If the distance L is greater than the predetermined distance Lth (step S164-No), the processor 21 executes, for example, the output control in the first seal mode (step S107) . On the other hand, if the distance L is the predetermined distance Lth or less, the processor 21 executes, for example, the output control in the second seal mode (step S108).

In the present modification, when the forceps 80 was detected in the observation image, the distance L between the forceps 80 (the region clamped by the forceps 80) and the grasping position of the blood vessel is calculated, and it is judged whether the distance L is the predetermined distance Lth or less. Thereby, it is properly judged whether the position of the detected forceps 80 is in the predetermined range from the grasping position of the blood vessel (the range of the predetermined distance Lth or less from the grasping position). Accordingly, in this modification, too, it is properly judged whether the forceps 80 exists in the predetermined range from the grasping position of the blood vessel.

Besides, each process illustrated in FIG. 3, FIG. 11, FIG. 14 and FIG. 15 may be executed by either the processor 21 of the control device (energy control device) 3 or the processor 72 of the image processing device 65. For example, in one modification, the processor 21 of the control device 3 executes the detection process of the forceps 80 in the set range (step S104). In another modification, the processor 72 of the image processing device 65 judges whether the forceps 80 exists in the predetermined range from the position where the blood vessel is grasped (the grasping position of the blood vessel) (step S106). In still another modification, an integral device having the functions of both the control device 3 and image processing device 65 may be provided in the processing system 1. In this modification, each process illustrated in FIG. 3, FIG. 11, FIG. 14 and FIG. 15 is executed by a processor provided in this integral device.

In the above-described embodiments, etc., an energy treatment instrument (2) of a treatment system (1) includes a first grasping piece (15), and a second grasping piece (16) configured to open and close relative to the first grasping piece (15) and configured to grasp a treated target between the first grasping piece (15) and the second grasping piece (16). In addition, in the energy treatment instrument (2), an actuation state is switched between a first mode in which the treated target is coagulated when a forceps (80) does not exist in a predetermined range from a position where the treated target is grasped, and a second mode in which the treated target is coagulated when the forceps (80) exists in the predetermined range. Besides, in the treatment system (1), an energy output source (32; 47; 32, 47) is configured to output electric energy which is supplied to the energy treatment instrument (2), and configured to apply treatment energy to the treated target which is grasped between the first grasping piece (15) and the second grasping piece (16), by the electric energy being supplied to the energy treatment instrument (2). An observation element (60) is configured to observe the treated target which is grasped. A processor (21, 72) is configured to judge whether the forceps (80) exists in the predetermined range from the position where the treated target is grasped, based on an observation image by the observation element (60). The processor (21, 72) is configured to execute at least one of controlling an output of the electric energy from the energy output source (32; 47; 32, 47), based on a judgement result of the forceps (80), and increasing a grasping force of the treated target between the first grasping piece (15) and the second grasping piece (16) in a case in which it was judged that the forceps (80) exists, compared to a case in which it was judged that the forceps (80) does not exist.

Although the embodiments, etc. of the present invention have been described above, the present invention is not limited to the above-described embodiments, etc., and, needless to say, various modifications can be made without departing from the spirit of the invention.

Hereinafter, characteristic items will be additionally described.

### (Additional Item 1)

A treatment method comprising:
closing a first grasping piece and a second grasping piece relative to each other, and grasping a treated target between the first grasping piece and the second grasping piece;
observing the treated target which is grasped;
supplying electric energy from an energy output source to the energy treatment instrument, and applying treatment energy to the treated target which is grasped between the first grasping piece and the second grasping piece;
judging whether a forceps exists in a predetermined range from a position where the treated target is grasped, based on an observation image of the treated target; and
executing at least one of controlling an output of the electric energy from the energy output source, based on a judgement result of the forceps, and increasing a grasping force of the treated target between the first grasping piece and the second grasping piece in a case in which it was judged that the forceps exists, compared to a case in which it was judged that the forceps does not exist.

## Claims

1. An energy treatment instrument comprising a first grasping piece, and a second grasping piece configured to open and close relative to the first grasping piece and configured to grasp a treated target between the first grasping piece and the second grasping piece,
wherein an actuation state is switched between a first mode in which the treated target is coagulated when a forceps does not exist in a predetermined range from a position where the treated target is grasped, and a second mode in which the treated target is coagulated when the forceps exists in the predetermined range.

2. The energy treatment instrument of Claim 1, wherein at least one of the first grasping piece and the second grasping piece includes an energy application section configured to apply treatment energy to the treated target which is grasped.

3. The energy treatment instrument of Claim 2, wherein an application state of the treatment energy from the energy application section to the treated target which is grasped is different between the first mode and the second mode.

4. The energy treatment instrument of Claim 1, wherein a grasping force of the treated target between the first grasping piece and the second grasping piece is different between the first mode and the second mode.

5. A treatment system comprising:
the energy treatment instrument of Claim 1;
an observation element configured to observe the treated target which is grasped;
an energy output source configured to output electric energy which is supplied to the energy treatment instrument, and configured to apply treatment energy to the treated target which is grasped between the first grasping piece and the second grasping piece, by the electric energy being supplied to the energy treatment instrument; and
a processor configured to judge whether the forceps exists in the predetermined range from the position where the treated target is grasped, based on an observation image by the observation element, and configured to control an output of the electric energy from the energy output source, based on a judgement result of the forceps, thereby switching the actuation state of the energy treatment instrument between the first mode and the second mode.

6. The treatment system of Claim 5, wherein the processor is configured to decrease the electric energy which is output, and increases an output time of the electric energy, in a case in which it was judged that the forceps exists, compared to a case in which it was judged that the forceps does not exist.

7. The treatment system of Claim 5, wherein the processor is configured to intermittently output the electric energy a plural number of times when it was judged that the forceps exists, by stopping the output of the electric energy after the output of the electric energy was started, and resuming the output of the electric energy after the output of the electric energy was once stopped.

8. The treatment system of Claim 5, wherein the processor is configured to detect an impedance between the first grasping piece and the second grasping piece, configured to stop, when it was judged that the forceps does not exist, the output of the electric energy, based on a fact that the impedance has increased to a first impedance threshold or more, and configured to stop, when it was judged that the forceps exists, the output of the electric energy, based on a fact that the impedance has increased to a second impedance threshold or more, which is greater than the first impedance threshold.

9. The treatment system of Claim 5, wherein the processor is configured to continuously stop the output of the electric energy, when it was judged that the forceps exists.

10. The treatment system of Claim 5, wherein the processor is configured to set as a detection range a range of a predetermined distance or less from the position where the treated target is grasped in the observation image, configured to detect the forceps in the detection range that is set, and configured to judge that the forceps exists in the predetermined range from the position where the treated target is grasped, when the forceps was detected in the detection range.

11. The treatment system of Claim 5, wherein the processor is configured to detect the forceps in an entirety of the observation image, configured to calculate, when the forceps was detected in the observation image, a distance between the detected forceps and the position where the treated target is grasped, and configured to judge that the forceps exists in the predetermined range from the position where the treated target is grasped, when the calculated distance is a predetermined distance or less.

12. The treatment system of Claim 5, wherein the first grasping piece includes a first electrode,
the second grasping piece includes a second electrode, and
the energy output source is configured to supply the output electric energy to the first electrode and the second electrode, thereby passing a high-frequency current as the treatment energy through the treated target between the first grasping piece and the second grasping piece.

13. A treatment system comprising:
the energy treatment instrument of Claim 1;
an observation element configured to observe the treated target which is grasped; and
a processor configured to judge whether the forceps exists in the predetermined range from the position where the treated target is grasped, based on an observation image by the observation element, and configured to increase a grasping force of the treated target between the first grasping piece and the second grasping piece in a case in which it was judged that the forceps exists, compared to a case in which it was judged that the forceps does not exist, thereby switching the actuation state of the energy treatment instrument between the first mode and the second mode.

14. A control device for use in combination with an energy treatment instrument including a first grasping piece, and a second grasping piece configured to open and close relative to the first grasping piece and configured to grasp a treated target between the first grasping piece and the second grasping piece,
the control device comprising:
an energy output source configured to output electric energy which is supplied to the energy treatment instrument, and configured to apply treatment energy to the treated target which is grasped between the first grasping piece and the second grasping piece, by the electric energy being supplied to the energy treatment instrument; and
a processor configured to judge whether a forceps exists in a predetermined range from a position where the treated target is grasped, based on an observation image observed by an observation element, and configured to execute at least one of controlling an output of the electric energy from the energy output source, based on a judgement result of the forceps, and increasing a grasping force of the treated target between the first grasping piece and the second grasping piece in a case in which it was judged that the forceps exists, compared to a case in which it was judged that the forceps does not exist.
